# EUROPEAN PATENT SPECIFICATION

(11) **EP 4 398 216 B1**
(45) Date of publication and mention of the grant of the patent: **19.08.2026**
(21) Application number: 22863873.0
(22) Date of filing: 09.03.2022
(51) Int. Cl.: G08G 1/16, G01C 21/26, G08B 25/00, G08B 25/01, G08B 25/10, A61B 5/02, G06V 20/59, H04M 11/06

(54) **NOTIFICATION DEVICE, NOTIFICATION METHOD, AND NOTIFICATION PROGRAM**
BENACHRICHTIGUNGSVORRICHTUNG, BENACHRICHTIGUNGSVERFAHREN UND BENACHRICHTIGUNGSPROGRAMM
DISPOSITIF DE NOTIFICATION, PROCÉDÉ DE NOTIFICATION ET PROGRAMME DE NOTIFICATION

(30) Priority: 02.09.2021 JP 2021143218
(43) Date of publication of application: 10.07.2024
(73) Proprietor: TRANSTRON Inc., Yokohama-shi, Kanagawa 222-0033 (JP)
(72) Inventor: OKAWA Ryota, Yokohama-shi, Kanagawa 222-0033 (JP)
(74) Representative: Winter, Brandl - Partnerschaft mbB
(86) International application number: PCT/JP2022/010399
(87) International publication number: WO 2023/032283

(56) References cited:
- WO-A1-2005/114576
- CN-Y- 201 114 324
- JP-A- 2017 228 280
- JP-A- 2018 206 197
- JP-A- H08 149 066
- JP-A- H1 023 230
- US-A1- 2008 037 837

## Description

### Technical Field

The present invention relates to a notification device, a notification method, and a notification program.

### Background Art

JP 2014-101 967 A discloses a method and a device for monitoring at least one occupant in a vehicle. The invention described in JP 2014-101 967 A uses at least one image capturing device to capture an image of a vehicle occupant and determines at least one vital sign of the vehicle occupant by analysis of the captured image.

US 2008 / 037 837 A discloses a notification device according to the preamble of claim 1. JP H10 23 230 A discloses further relevant background information.

JP H10 23230 relates to a vehicle-mounted facsimile modem to transmit analog FAX data (image data) via a voice modem circuit.

### Summary of Invention

### Technical Problem

In the invention described in Patent Literature 1, although it is described that the result of image analysis is provided at the time of medical care in an emergency situation, it is unclear in what form the result is provided. For example, it can be said that it is effective to transmit the image when the occupant is alive but cannot talk, but the image cannot be transmitted unless a specification of a voice communication system used for emergency notification is changed, which is not easy.

The present invention has been made in view of such circumstances, and an object thereof is to provide a notification device, a notification method, and a notification program that allow transmitting image information of an inside of a vehicle interior using an existing voice communication system.

### Solution to Problem

In order to solve the problem, a notification device according to the present invention is, for example, a notification device mounted on a vehicle. The notification device includes an imaging unit, a conversion unit, and a communication unit. The imaging unit is provided in the vehicle and acquires a planar image of an inside of a vehicle interior of the vehicle. The conversion unit converts the planar image into an audio signal in the voice band to generate a first audio signal. The communication unit is provided in the vehicle and transmits the first audio signal.

In order to solve the problem, a notification method according to the present invention, for example, includes: using the notification device for a planar image of an inside of a vehicle interior of a vehicle; converting the planar image into an audio signal in the voice band to generate a first audio signal; and transmitting the first audio signal.

In order to solve the problem, a notification method according to the present invention, for example, is for causing a computer in the notification device to function as: a conversion unit that converts a planar image of an inside of a vehicle interior imaged by an imaging unit provided in a vehicle into an audio signal in the voice band to generate a first audio signal; and a communication unit that transmits the first audio signal.

Note that the computer program can be provided by being downloaded via a network such as the Internet, or can be provided by being recorded in various computer-readable recording media such as a CD-ROM.

In any of the aspects of the present invention, the planar image of the inside of the vehicle interior is acquired by the imaging unit provided in the vehicle, and the planar image is converted into the audio signal to generate and transmit the first audio signal. Thus, image information of the inside of the vehicle interior can be transmitted using an existing voice communication system. Therefore, it is not necessary to change specifications of a communication facility including a TCU and a communication infrastructure side. In addition, information of the inside of the vehicle interior can be grasped even when an occupant cannot make a voice call.

An image transmission trigger detection unit that detects an image transmission trigger requesting transmission of the planar image may be provided. The conversion unit may generate the first audio signal when the image transmission trigger is detected. Thus, the audio signal generated by converting the planar image can be transmitted only when necessary. Since the first audio signal generated by converting the planar image is heard like noise, there is a possibility that the first audio signal interferes with a call. However, since the first audio signal is transmitted only when necessary, the first audio signal does not interfere with a call.

The image transmission trigger may be a sound. The communication unit may receive the sound and output the sound to the image transmission trigger detection unit. The image transmission trigger detection unit may determine that the image transmission trigger is detected when the sound is detected. In this way, by transmitting the first audio signal to an external device in response to an instruction from the external device (for example, a center-side device), the external device that has received the audio signal easily recognizes a timing at which the first audio signal has been transmitted, and easily creates a spectrogram based on the first audio signal.

A microphone provided inside of the vehicle interior may be provided. The image transmission trigger detection unit may detect an utterance from a voice acquired by the microphone, and determine that the image transmission trigger is detected when an utterance is not detected for a predetermined time. Accordingly, the notification device can automatically generate and transmit the first audio signal in a situation where an occupant cannot make a voice call (fainting or the like).

The conversion unit may configure the planar image in which real numbers are two-dimensionally arrayed as a spectrogram, add phase information to the spectrogram to generate complex number data in which complex numbers are two-dimensionally arrayed, and perform inverse Fourier transform on the complex number data to generate the first audio signal. The conversion unit may randomly generate the phase information using a random number. Thus, the first audio signal can be generated from the planar image. Further, by using random numbers, the phase information can be added as appropriate.

A time-series data acquisition unit that acquires time-series data may be provided. The conversion unit may convert the time-series data into an audio signal in the voice band to generate a second audio signal. The communication unit may transmit the second audio signal. The conversion unit may perform analog modulation on the time-series data such that a frequency range falls within a voice band to generate the second audio signal. This makes it possible to transmit not only the planar image (two-dimensional data) but also the time-series data (one-dimensional data) using an existing voice communication system.

The time-series data acquisition unit may be a biological information acquisition unit that acquires biological information including at least one of a heart rate, a respiratory frequency, a blood pressure, a blood sugar level, a body temperature, and an arterial oxygen saturation. The conversion unit may convert the biological information into an audio signal in the voice band to generate the second audio signal. This makes it possible to transmit not only the planar image but also the biological information using an existing voice communication system.

A biological information acquisition unit that acquires biological information including at least one of a heart rate, a respiratory frequency, a blood pressure, a blood sugar level, a body temperature, and an arterial oxygen saturation may be provided. When the biological information is smaller than a first threshold or larger than a second threshold larger than the first threshold, the image transmission trigger detection unit may determine that the image transmission trigger is detected. Accordingly, the notification device can automatically generate and transmit the first audio signal in a situation where an occupant cannot make a voice call (fainting or the like).

The imaging unit may continuously image the planar image. The conversion unit may continuously generate the first audio signal. As a result, the external device or the like can more reliably grasp a change in the situation inside the vehicle interior.

### Advantageous Effects of Invention

The present invention allows transmitting the image information of the inside of the vehicle interior using an existing voice communication system.

### Brief Description of Drawings

FIG. 1 is a diagram schematically illustrating a vehicle 10 including a notification device 1 according to a first embodiment and a center-side device 20 disposed in a call center.
FIG. 2 is an example of a spectrogram.
FIG. 3 is a diagram schematically illustrating a flow of processes for generating the spectrogram.
FIG. 4 is a block diagram schematically illustrating an example of a hardware configuration of the notification device 1.
FIG. 5 is a block diagram illustrating an outline of a functional configuration of the notification device 1.
FIG. 6 is a diagram schematically illustrating processes performed by a first conversion unit 102a.
FIG. 7 is a diagram schematically illustrating processes performed by the first conversion unit 102a.
FIG. 8(A) is an example of a planar image P1, and FIG. 8(B) is an example of an audio signal in the voice band A1 and a spectrogram |X(m, k)|.
FIG. 9 is a flowchart depicting a flow of processes performed by the notification device 1.
FIG. 10 is a flowchart depicting a flow of processes in a modification of the notification device 1.
FIG. 11 is a block diagram illustrating an outline of a functional configuration of a notification device 2.
FIG. 12 is a flowchart depicting a flow of processes performed by the notification device 2.
FIG. 13 is a block diagram illustrating an outline of a functional configuration of a notification device 3.
FIG. 14 is a flowchart depicting a flow of processes performed by the notification device 3.

### Description of Embodiments

Hereinafter, with reference to the drawings, detailed description is made on a notification device according to embodiments of the present invention. The notification device of the present invention is a device mounted on a vehicle, and starting a voice conversation by notifying a call center from a vehicle. Hereinafter, the present invention will be described with an example of an emergency notification device that automatically notifies a call center when an accident, such as deployment of an airbag, occurs or when an emergency notification button is pressed, and the like, but the notification device of the present invention is not limited to the emergency notification device.

### First Embodiment

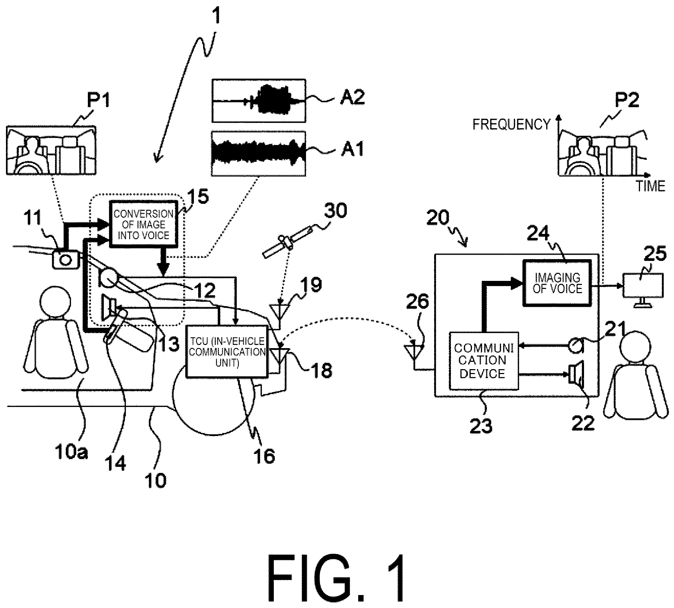

FIG. 1 is a diagram schematically illustrating a vehicle 10 including a notification device 1 according to the first embodiment and a center-side device 20 disposed in a call center. The notification device 1 is connected to the center-side device 20 wirelessly or over a network (in this embodiment, a communication network for mobile phones). The notification device 1 mainly includes an imaging unit 11, a microphone 12, a speaker 13, a biological information acquisition unit 14, a processing unit 15 that performs, for example, a process for converting an image into a voice, a telematics control unit (TCU) 16, and antennas 18 and 19.

The imaging unit 11, the microphone 12, and the speaker 13 are provided inside of a vehicle interior 10a of the vehicle 10. The imaging unit 11 is, for example, a visible light camera, such as an RGB camera that images a color image or a monochrome camera, or an infrared camera or a thermography that detects electromagnetic waves in an infrared wavelength region and converts them into an image, and acquires a planar image P1 of the inside of the vehicle interior 10a.

The biological information acquisition unit 14 is, for example, a heart rate meter provided on a steering wheel, and acquires a heart rate of a driver. The biological information acquisition unit 14 is not limited to the heart rate meter and only needs to acquire biological information including at least one of a heart rate, a respiratory frequency, a blood pressure, a blood sugar level, a body temperature, and an arterial oxygen saturation.

The processing unit 15 converts the planar image P1 (two-dimensional data) into an audio signal in the voice band to generate an audio signal A1. In addition, the processing unit 15 converts the biological information (one-dimensional data) acquired by the biological information acquisition unit 14 into an audio signal in the voice band to generate an audio signal A2. The process of converting the planar image P1 and the biological information into the audio signals and the like will be described in detail later.

The TCU 16 controls bidirectional communications in a call, Electronic Toll Collection System (ETC), and the like. The TCU 16 transmits the audio signals A1 and A2 and the voice signal input from the microphone 12 to the center-side device 20 via the antenna 18.

The processing unit 15 and the TCU 16 may be constructed as, for example, a dedicated board mounted on an in-vehicle device provided in the vehicle 10.

The antenna 18 receives radio waves transmitted from the center-side device 20 via the network for mobile phones, and transmits the radio waves to the center-side device 20. The antenna 19 receives radio waves transmitted from a Global Navigation Satellite System (GNSS).

The center-side device 20 mainly includes a microphone 21, a speaker 22, a communication device 23, a processing unit 24 that performs processes for imaging a voice, a display unit 25, and an antenna 26.

The communication device 23 receives radio waves transmitted from the notification device 1 via the antenna 26 and the network for mobile phones, and transmits radio waves to the notification device 1. The communication device 23 is connected to the microphone 21, the speaker 22, and the processing unit 24, a voice signal is input from the microphone 21 to the communication device 23, and the communication device 23 outputs the voice signal to the speaker 22 and the processing unit 24. The voice signal input from the microphone 12 is output to the speaker 22, and the audio signals A1 and A2 are output to the processing unit 24.

The processing unit 24 generates a spectrogram P2 based on the input voice signal A1, and acquires the biological information based on the input voice signal A2. The spectrogram P2 is output from the processing unit 24 to the display unit 25 and is displayed on the display unit 25. The spectrogram represents results obtained by performing frequency analysis continuously temporally by a two-dimensional graph (a time axis and a frequency axis).

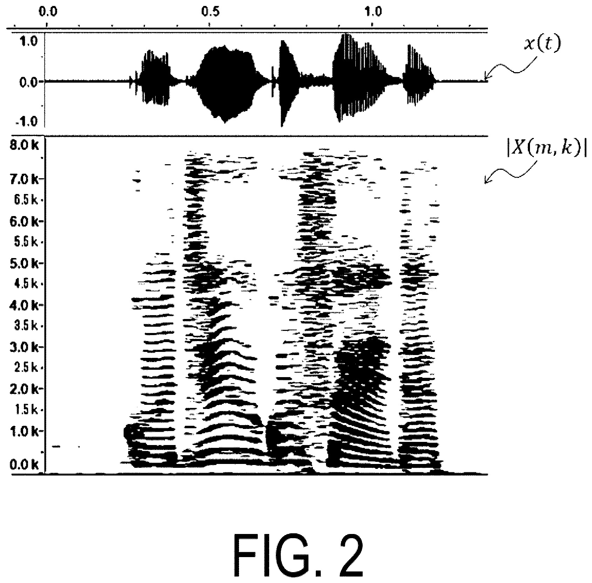

FIG. 2 is an example of the spectrogram. The upper part of FIG. 2 shows an audio signal in the voice band x(t), the horizontal axis represents time, and the vertical axis represents amplitude. Note that (t) represents a function of time. The lower part of FIG. 2 is a spectrogram |X(m, k)| in which the horizontal axis represents a time, the vertical axis represents a frequency, and a density of a color at each point represents a magnitude (amplitude) at a certain frequency at a certain time point. Note that m is a frame number and k is a discrete frequency number. Note that the spectrogram display method is not limited to the two-dimensional graph, and may be a three-dimensional graph (waterfall plot).

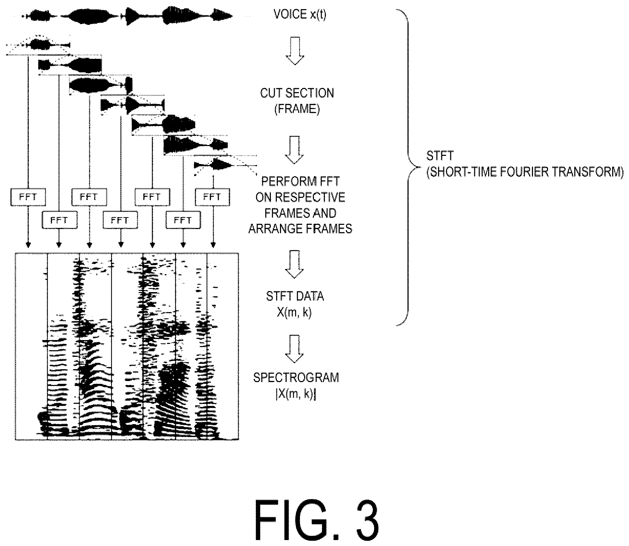

FIG. 3 is a diagram schematically illustrating a flow of processes for generating the spectrogram. First, a window function (see the dotted line in FIG. 3) is integrated into the audio signal x(t) and a part of the audio signal x(t) is cut. Hereinafter, the cut section is referred to as a frame. At this time, an overlap process is applied to overlap a part of the previous frame with a part of the current frame.

Next, fast Fourier transform (FFT) is performed on each frame, and the calculation results are arranged along the time axis. As a result, a two-dimensional array of complex numbers is obtained. Hereinafter, the two-dimensional array obtained here is referred to as STFT data X(m, k).

Since the STFT data X(m, k) is complex numbers, the absolute value |X(m, k)| of the STFT data or the power |X(m, k)|² of the STFT data is obtained for conversion into real numbers. In the present embodiment, the spectrogram |X(m, k)| is obtained by acquiring the two-dimensional array of the absolute values |X(m, k)| of the STFT data.

Although the spectrogram is drawn as a monochrome image in FIGS. 2 and 3, the spectrogram may be displayed as a color image. Further, the density of the color of the spectrogram may be logarithmically converted (dB conversion) and displayed.

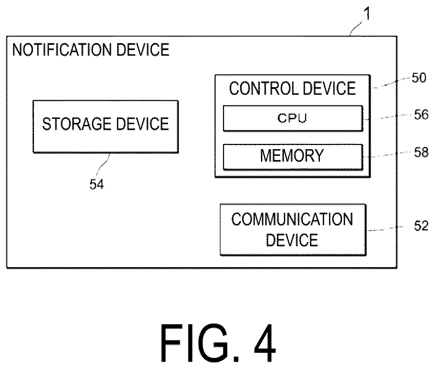

FIG. 4 is a block diagram schematically illustrating an example of a hardware configuration of the notification device 1. The notification device 1 includes a control device 50, a communication device 52, and a storage device 54.

The control device 50 mainly includes a Central Processing Unit (CPU) 56 and a memory 58. In the control device 50, the CPU 56 executes predetermined programs stored in the storage device 54, the memory 58, or the like to function as various functional units. Note that the programs may be temporarily or permanently stored (memorized) in a removable storage medium, such as a semiconductor memory, a memory card, an optical disc, a magneto-optical disk, and a magnetic disk.

The communication device 52 includes a communication interface or the like for communicating with an external device. The communication device 52 transmits and receives various pieces of information to and from, for example, the center-side device 20. The storage device 54 is formed by a hard disk or the like. The storage device 54 stores various programs and various pieces of information required for the control device 50 to perform the process and information on results of the process.

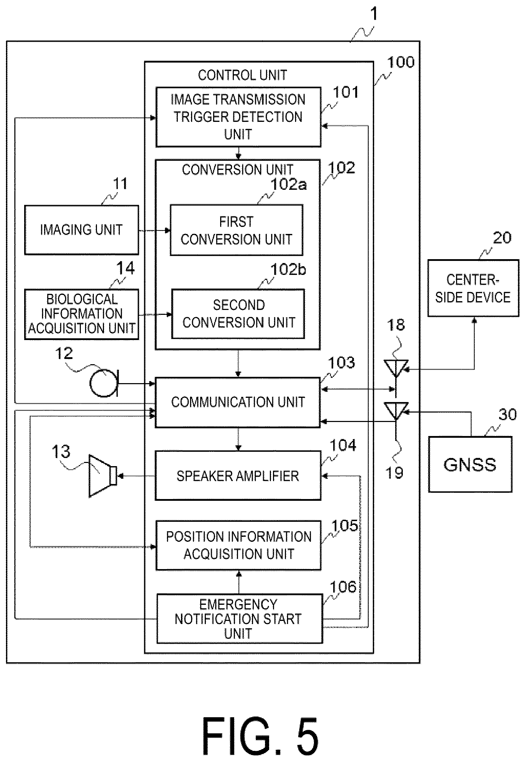

FIG. 5 is a block diagram illustrating an outline of a functional configuration of the notification device 1. The notification device 1 functionally includes a control unit 100. The control unit 100 includes at least an image transmission trigger detection unit 101, a conversion unit 102, a communication unit 103, a speaker amplifier 104, a position information acquisition unit 105, and an emergency notification start unit 106 as software resources by an arithmetic device, such as the Central Processing Unit (CPU) 56 for executing information processing and the storage device 54.

The image transmission trigger detection unit 101 is a functional unit that detects the image transmission trigger requesting transmission of an image. In the present embodiment, the image transmission trigger is a sound, and, for example, a sound in which 440 Hz and 880 Hz sequentially continue for 0.5 seconds is used as the image transmission trigger. Note that the form of the image transmission trigger is not limited to this and may be, for example, a voice, such as "Please send the image."

The image transmission trigger is transmitted from the center-side device 20 and received by the communication unit 103, and is output from the communication unit 103 to the image transmission trigger detection unit 101. The image transmission trigger detection unit 101 detects the image transmission trigger by acquiring a sound indicating the image transmission trigger in advance and comparing the sound acquired in advance with the voice output from the communication unit 103.

The conversion unit 102 mainly includes a first conversion unit 102a that converts the planar image P1 imaged by the imaging unit 11 into an audio signal in the voice band to generate the audio signal A1, and a second conversion unit 102b that converts the biological information acquired by the biological information acquisition unit 14 into an audio signal in the voice band to generate the audio signal A2.

The first conversion unit 102a is a functional unit that configures the planar image P1 in which real numbers are two-dimensionally arrayed (two-dimensional data) as a spectrogram, adds phase information to the spectrogram to generate complex number data (STFT data X(m, k)) in which complex numbers are two-dimensionally arrayed, and performs inverse Fourier transformation on the complex number data to generate the audio signal A1.

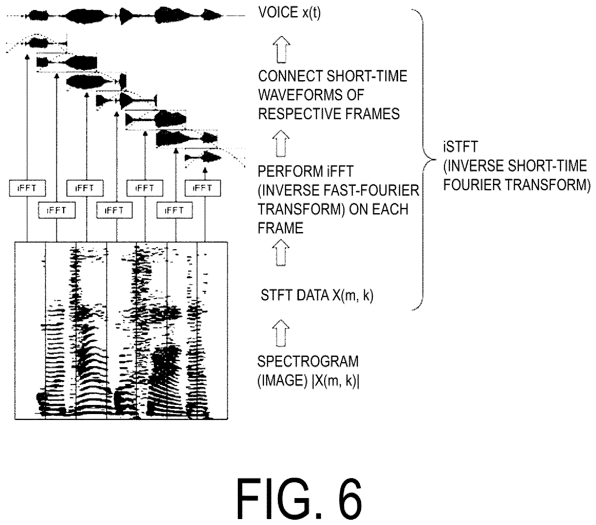

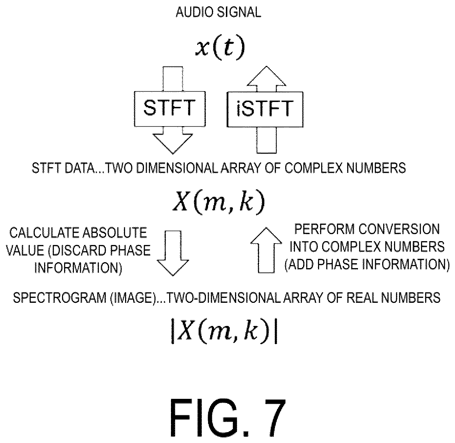

FIGS. 6 and 7 are diagrams schematically illustrating processes performed by the first conversion unit 102a. Specifically, as illustrated in FIG. 6, the first conversion unit 102a first adds the phase information to the planar image P1 and converts it into complex numbers to generate the STFT data X(m, k), performs inverse short-time Fourier transformation (iSTFT, inverse STFT), and generates the audio signal x(t) by connecting them.

As illustrated in FIG. 7, the inverse short-time Fourier transform is an inverse transform of the short-time Fourier transform, and the audio signal x(t) and the STFT data X(m, k) can be mutually converted by performing the short-time Fourier transform and the inverse short-time Fourier transform.

Since the absolute value is obtained, in the conversion from the STFT data X(m, k) into the spectrogram |X(m, k)|, the phase information may be discarded. On the other hand, in the conversion from the spectrogram |X(m, k)| into the STFT data X(m, k), it is necessary to add the phase information and perform conversion into complex numbers. In the present embodiment, the phase information is randomly generated using a random number as shown in the following Equation 1. $X \left(m, k\right) = \left|X\left(m, k\right)\right| {e}^{jθ \left(m, k\right)}$
θ(m, k): uniform random number from -π to +π
j: imaginary unit

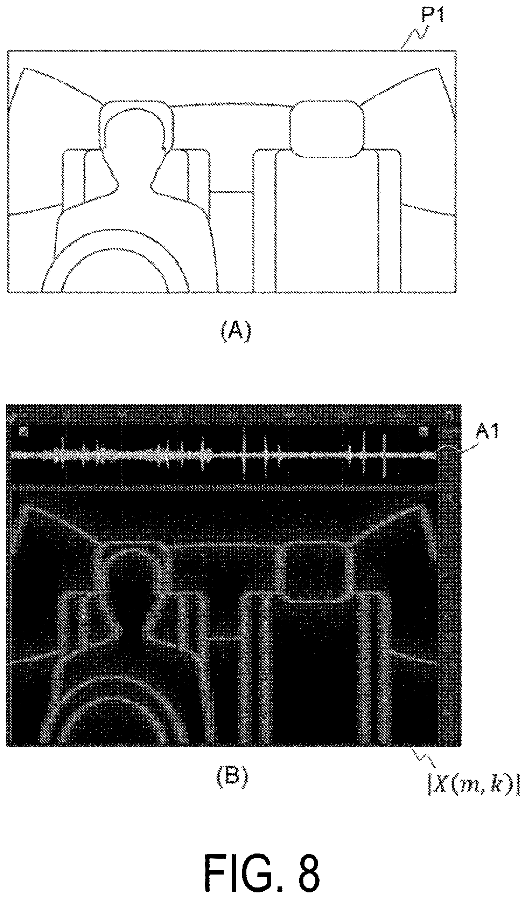

FIG. 8(A) is an example of the planar image P1, and FIG. 8(B) is an example of the audio signal A1 and the spectrogram |X(m, k)|. The upper part of FIG. 8(B) is the audio signal A1, and the lower part of FIG. 8(B) is the spectrogram |X(m, k)|. In the spectrogram |X(m, k)|, the contour line of the planar image P1 is clearly illustrated, and the same effect as that of viewing the planar image P1 can be obtained by viewing the spectrogram |X(m, k)|.

The description will now return to FIG. 5. The second conversion unit 102b is a functional unit that converts biological information, which is a time-series signal (one-dimensional signal) in which real numbers are arranged in time series, into an audio signal in the voice band to generate the audio signal A2. The audio signal A2 can be generated by, for example, analog modulation, such as FM modulation, AM modulation, or PM modulation. However, a frequency after modulation needs to fall within a voice band of emergency notification. For example, when a narrow-band voice codec is used in communication, when the FM modulation is performed with frequencies of FM carriers as 2 kHz and a maximum frequency shift as 1 kHz, the frequency range becomes 1 kHz to 3 kHz and falls within the voice band of 0. 3 kHz to 3. 4 kHz.

Note that the audio signal A2 is not limited to be generated by analog modulation. For example, the biological information may be digitized into binary data, and the audio signal A2 may be generated by digital modulation, such as quadrature amplitude modulation (QAM).

The communication unit 103 is a functional unit that transmits and receives signals via the antennas 18 and 19. When the audio signals A1 and A2 are generated by the conversion unit 102, the communication unit 103 transmits the audio signals A1 and A2. Further, the communication unit 103 transmits a voice signal input from the microphone 12.

Further, the communication unit 103 receives a voice transmitted from the center-side device 20. The communication unit 103 outputs the received voice to the image transmission trigger detection unit 101 and the speaker amplifier 104.

The communication unit 103 receives radio waves transmitted from a GNSS 30 via the antenna 19, and outputs the radio waves to the position information acquisition unit 105.

The speaker amplifier 104 is a functional unit that amplifies a signal output from the communication unit 103 and outputs the amplified signal to the speaker 13. The position information acquisition unit 105 is a functional unit that acquires radio waves transmitted from the GNSS 30 via the communication unit 103 and acquires position information based on the acquired radio waves. Since the speaker amplifier 104 and the position information acquisition unit 105 are already known, description thereof is omitted.

The emergency notification start unit 106 is a functional unit that starts emergency notification when an emergency notification start trigger is input. The emergency notification start trigger is input from an in-vehicle device (not illustrated) or the like. The emergency notification start unit 106 is connected to the image transmission trigger detection unit 101, the communication unit 103, the speaker amplifier 104, and the position information acquisition unit 105, and outputs an instruction to start the emergency notification to these functional units when the emergency notification start trigger is input.

Note that FIG. 4 only illustrates a part of the main hardware configuration provided with the notification device 1, and the notification device 1 can include other configurations that are generally included in a server device. The notification device 1 can be achieved using an information processing device, such as a dedicated or general-purpose server computer. The notification device 1 may be configured by a single information processing device, or may be configured by a plurality of information processing devices distributed on a communication network. The functional components of the notification device 1 illustrated in FIG. 5 may be classified into more components according to the processing content, or one component may perform processes of a plurality of components.

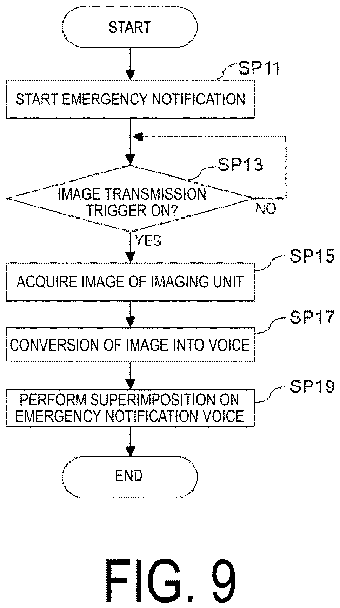

FIG. 9 is a flowchart depicting a flow of processes performed by the notification device 1. First, when the emergency notification start trigger is input from the in-vehicle device or the like, the emergency notification start unit 106 outputs an emergency notification start instruction to the image transmission trigger detection unit 101, the communication unit 103, the speaker amplifier 104, and the position information acquisition unit 105, and starts the emergency notification (Step SP11).

Specifically, the communication unit 103 receives a voice or the like from the center-side device 20 via the antenna 18 based on the emergency notification start instruction, and outputs the voice or the like to the speaker amplifier 104. Thus, the voice output from the center-side device 20 is output from the speaker 13. In addition, the communication unit 103 outputs the voice (emergency notification voice) input from the microphone 12 to the center-side device 20 via the antenna 18 based on the emergency notification start instruction. Thus, the emergency notification voice can be heard by the center-side device 20. When the emergency notification start instruction is input, the image transmission trigger detection unit 101 waits for the input of the image transmission trigger. These processes are continuously performed while the emergency notification is being performed.

In addition, the position information acquisition unit 105 receives the radio wave from the GNSS 30 via the antenna 19 based on the emergency notification start instruction, and outputs the radio wave to the position information acquisition unit 105. The position information acquisition unit 105 obtains the position information and outputs the position information to the communication unit 103. The communication unit 103 outputs the position information to the center-side device 20 via the antenna 18. The position information acquisition unit 105 or the transmission of the position information is not essential.

Next, the image transmission trigger detection unit 101 determines whether or not the image transmission trigger has been input via the communication unit 103 (Step SP13).

When the image transmission trigger is not input (NO in Step SP13), the image transmission trigger detection unit 101 repeats the process in Step SP13. When the image transmission trigger is input (YES in Step SP13), the image transmission trigger detection unit 101 outputs that the image transmission trigger is input to the conversion unit 102, and the first conversion unit 102a acquires the planar image P1 from the imaging unit 11 (Step SP15).

Next, the first conversion unit 102a converts the planar image P1 acquired in Step SP13 into a voice to generate the audio signal A1 (Step SP17). The first conversion unit 102a outputs the generated voice signal A1 to the communication unit 103. The communication unit 103 superimposes the audio signal A1 on the emergency notification voice and transmits it (Step SP19).

Thereafter, the emergency notification start unit 106 ends the series of processes. When the emergency notification start trigger is input from the in-vehicle device or the like while the process depicted in FIG. 9 is being performed, the emergency notification start unit 106 ends the process depicted in FIG. 9.

The center-side device 20 receives the audio signal A1 transmitted from the notification device 1 in this way. As illustrated in FIG. 1, the processing unit 24 generates the spectrogram P2 based on the input voice signal A1. As a result, the spectrogram P2 is displayed on the display unit 25 of the center-side device 20, and a state of the inside of the vehicle interior 10a can be confirmed at the call center.

When the image transmission trigger is input from the image transmission trigger detection unit 101 to the conversion unit 102, the second conversion unit 102b may acquire the biological information from the biological information acquisition unit 14 at the same time as Step SP13, the second conversion unit 102b may convert the biological information into a voice to generate the audio signal A2 at the same time as Step SP17, and the communication unit 103 may superimpose the audio signal A2 on the emergency notification voice and transmit it at the same time as Step SP19.

According to the present embodiment, the plane image P1 imaged by the imaging unit 11 is converted into the audio signal to generate the audio signal A1, and when the audio signal A1 is generated, the audio signal A1 is transmitted such that the image information of the inside of the vehicle interior can be transmitted using the existing voice communication system. Therefore, it is not necessary to change specifications of a communication facility including the TCU 16 and a communication infrastructure side. Since the TCU 16 relays a notification voice and does not have an image transmission function, to transmit an image via the TCU 16, an interface with the imaging unit 11 and firmware for image transmission need to be provided, which takes labor and cost. On the other hand, when the audio signal A1 obtained by converting the planar image is transmitted as in the present embodiment, it is not necessary to change the TCU 16, labor or cost is not taken, and it is possible to add an image communication function to a vehicle on which the TCU 16 not having the image transmission function is mounted afterward.

Further, according to the present embodiment, by transmitting the audio signal A1 obtained by converting the planar image P1, information of the inside of the vehicle interior 10a can be grasped even when an occupant cannot make a voice call.

Further, according to the present embodiment, when the image transmission trigger transmitted from the center-side device 20 is detected by the image transmission trigger detection unit 101, the plane image P1 is converted into a voice by the first conversion unit 102a to generate the audio signal A1, and therefore the audio signal A1 can be transmitted only when necessary. When the audio signal A1 is transmitted at all times, the audio signal A1 is heard as noise, which possibly interferes with a call. On the other hand, by transmitting the audio signal A1 using the image transmission trigger as a trigger as in the present embodiment, the audio signal A1 can be transmitted as necessary without interfering with a call. In particular, in the present embodiment, since the image transmission trigger transmitted from the center-side device 20 is used as the trigger for transmission of the audio signal A1, the timing at which the audio signal A1 is transmitted by the center-side device 20 is easily understood, and a spectrogram is easily created based on the audio signal A1.

Further, according to the present embodiment, the image transmission trigger from the center-side device 20 is configured as a sound, and therefore the image transmission trigger can be superimposed on a call voice. Therefore, the image transmission trigger can be transmitted from the center-side device 20 without changing the specification of the TCU 16.

Further, according to the present embodiment, the biological information acquired by the biological information acquisition unit 14 is converted into the audio signal to generate the audio signal A2, and therefore not only the planar image P1 but also the biological information can be transmitted using the existing voice communication system.

In the present embodiment, the notification device 1 includes the microphone 12, the speaker 13, and the speaker amplifier 104 to bidirectionally communicate with the center-side device 20, but the microphone 12, the speaker 13, or the speaker amplifier 104 is not essential. To transmit the audio signal A1, the notification device 1 only needs to include at least the image transmission trigger detection unit 101, the conversion unit 102, and the communication unit 103.

In addition, generation or transmission of the audio signal A1 based on the image transmission trigger detection unit 101, that is, the image transmission trigger, is not essential. For example, the notification device 1 may generate and transmit the audio signal A1 to start notification. However, transmission of the audio signal A1 only when necessary based on the image transmission trigger is desirable so as not to interfere with a call.

In the present embodiment, the biological information acquired by the biological information acquisition unit 14 is converted into the audio signal A2 by the second conversion unit 102b and transmitted from the communication unit 103, but the biological information acquisition unit 14 or the second conversion unit 102b is not essential. For example, the notification device 1 may acquire the biological information from a device other than the biological information acquisition unit 14, for example, a wearable device via the communication unit 103.

In addition, in the present embodiment, the notification device 1 converts the biological information acquired by the biological information acquisition unit 14 into the audio signal A2 and transmits the audio signal A2 from the communication unit 103. However, the notification device may transmit various forms of time series data other than the biological information as an audio signal in the voice band. For example, the communication device may include at least one of a temperature sensor, a humidity sensor, and an atmospheric pressure sensor, acquire temperature data, humidity data, and atmospheric pressure data as time-series data from the temperature sensor, the humidity sensor, and the atmospheric pressure sensor, respectively, convert them into voice signals by the second conversion unit 102b, and transmit the audio signals from the communication unit 103. In addition, for example, the communication device may include a gas sensor (for example, a CO₂ sensor), acquire a substance concentration data (for example, carbon dioxide concentration data), which is the time-series data, from the gas sensor, convert it into an audio signal in the voice band by the second conversion unit 102b, and transmit the audio signal from the communication unit 103. The time-series data, such as the temperature data, the humidity data, the atmospheric pressure data, and the substance concentration data, can also be converted into an audio signal in the voice band by the method similar to that for biological information.

In addition, in the present embodiment, in Step SP15, the communication unit 103 superimposes the audio signal A1 on the emergency notification voice and transmits it, but the communication unit 103 may transmit only the audio signal A1.

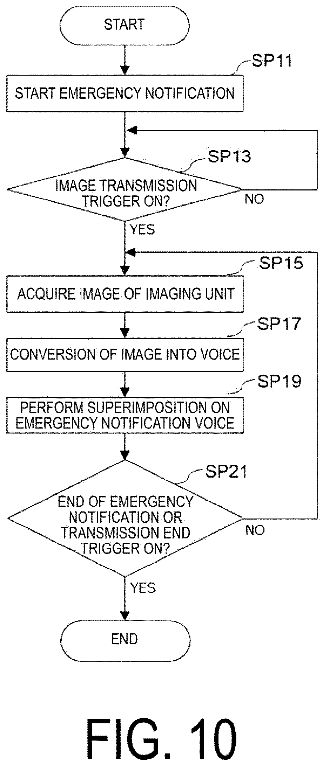

In addition, in the present embodiment, the audio signal A1 is transmitted only once and the process ends, but a plurality of the audio signals A1 may be transmitted. FIG. 10 is a flowchart depicting a flow of processes in a modification of the notification device 1. Since Steps SP11 to SP19 are the same, description thereof will be omitted.

When the audio signal A1 is superimposed on the emergency notification voice and transmitted (Step SP19), the image transmission trigger detection unit 101 determines whether or not the emergency notification is ended or the transmission end trigger is input via the communication unit 103 (Step SP21). For example, the image transmission trigger detection unit 101 determines that the emergency notification is ended when a signal indicating the end of the emergency notification is output from the in-vehicle device or the like, is input to the emergency notification start unit 106, and is input from the emergency notification start unit 106 to the image transmission trigger detection unit 101. Further, for example, the image transmission trigger detection unit 101 determines that the transmission end trigger is input when the transmission end trigger is transmitted from the center-side device 20 and is input to the image transmission trigger detection unit 101 via the communication unit 103. The image transmission trigger detection unit 101 acquires a sound indicating the transmission end trigger in advance, and detects the transmission end trigger by comparing the sound acquired in advance with the voice output from the communication unit 103. For example, a sound in which 440 Hz and 880 Hz sequentially continue for one second can be used as the transmission end trigger, but the form of the transmission end trigger is not limited to this.

When the emergency notification is not ended or the transmission end trigger is not input (NO in Step SP21), the image transmission trigger detection unit 101 returns the process to Step SP15. That is, until the transmission end trigger is input, processes of the first conversion unit 102a acquiring the planar image P1 (Step SP15) and converting the planar image P1 into a voice to generate the audio signal A1 (Step SP17), and the communication unit 103 superimposing the audio signal A1 on the emergency notification voice to transmit it (Step SP19) are continued.

When the emergency notification is ended or the transmission end trigger is input (YES in Step SP21), the emergency notification start unit 106 ends the series of processes. When the emergency notification start trigger is input from the in-vehicle device or the like while the process depicted in FIG. 10 is being performed, the emergency notification start unit 106 ends the process depicted in FIG. 10.

According to the present modification, since the audio signal A1 can be continuously transmitted, the center-side device 20 can more reliably grasp the change in the situation inside the vehicle interior 10a.

### Second Embodiment

In the first embodiment of the present invention, when the image transmission trigger transmitted from the center-side device 20 is input to the image transmission trigger detection unit 101 via the communication unit 103, the first conversion unit 102a converts the plane image P1 into a voice to generate the audio signal A1, and the communication unit 103 superimposes the audio signal A1 on the emergency notification voice and transmits it, but the trigger for generating the audio signal A1 from the plane image P1 is not limited to this.

The second embodiment of the present invention is an embodiment in which the audio signal A1 is generated from the planar image P1 when no utterance by a person in the vehicle interior 10a is detected as a trigger. Hereinafter, description is made on a notification device 2 according to the second embodiment of the present invention. Note that the same components as those in the first embodiment are denoted by the same reference numerals, and descriptions thereof will be omitted.

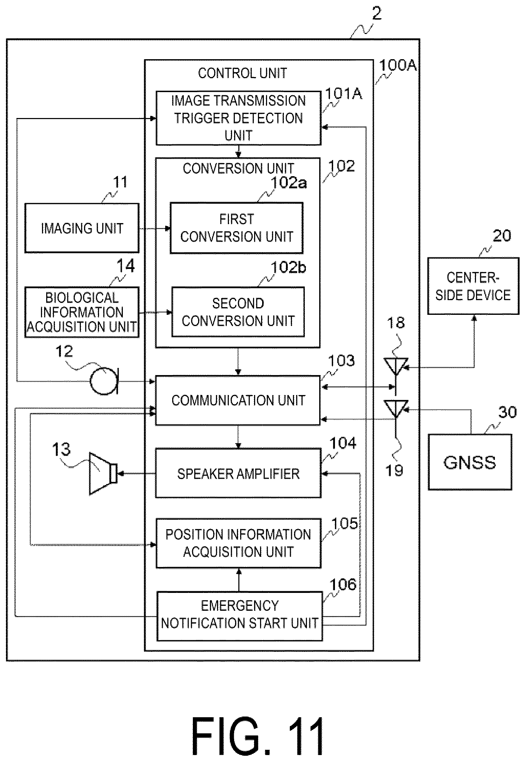

FIG. 11 is a block diagram illustrating an outline of a functional configuration of the notification device 2. Similarly to the notification device 1, the notification device 2 includes the control device 50, the communication device 52, and the storage device 54 as a hardware configuration. The notification device 2 functionally includes a control unit 100A.

The control unit 100A includes at least an image transmission trigger detection unit 101A, the conversion unit 102, the communication unit 103, the speaker amplifier 104, the position information acquisition unit 105, and the emergency notification start unit 106 as software resources by an arithmetic device, such as the Central Processing Unit (CPU) 56 for executing information processing, and the storage device 54.

The image transmission trigger detection unit 101A is a functional unit that detects the image transmission trigger requesting transmission of an image. The voice acquired by the microphone 12 is input to the image transmission trigger detection unit 101A. For example, the image transmission trigger detection unit 101A determines that the image transmission trigger is detected when an utterance is detected from the voice acquired by the microphone 12, and an utterance is not detected for a predetermined time. The predetermined time is, for example, 10 seconds, and any time can be set.

As described above, in the case where the notification device 2 cannot detect an utterance for the predetermined time, the notification device 2 can automatically determine that the occupant cannot make a voice call (fainting, death, or the like) and a timing to transmit the image of the inside of the vehicle interior 10a has come.

Various known methods can be used as a method of detecting an utterance from the voice acquired by the microphone 12. In the present embodiment, the image transmission trigger detection unit 101A determines that there is an utterance when a sound pressure level acquired by the microphone 12 exceeds a threshold, and determines that there is no utterance when the sound pressure level does not exceed the threshold.

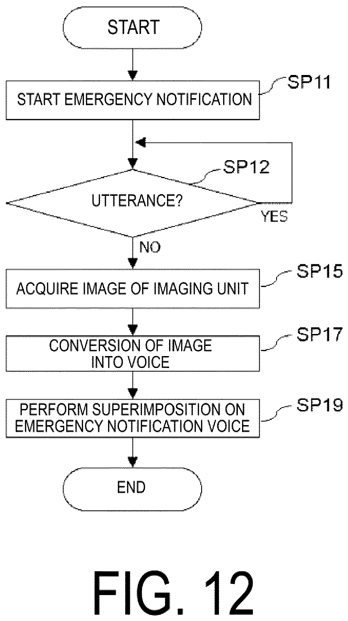

FIG. 12 is a flowchart depicting a flow of processes performed by the notification device 2. First, when the emergency notification start trigger is input, the emergency notification start unit 106 outputs the emergency notification start instruction to the image transmission trigger detection unit 101A, the communication unit 103, the speaker amplifier 104, and the position information acquisition unit 105, and starts the emergency notification (Step SP11).

Next, the image transmission trigger detection unit 101A detects an utterance from the voice acquired by the microphone 12, and determines whether or not the utterance is detected for a predetermined time (Step SP12).

When an utterance is detected (YES in Step SP12), the image transmission trigger detection unit 101A repeats the process of Step SP12. When an utterance is not detected for the predetermined time (NO in Step SP12), the image transmission trigger detection unit 101A outputs that the image transmission trigger is input to the conversion unit 102, and the first conversion unit 102a acquires the planar image P1 from the imaging unit 11 (Step SP15).

Next, the first conversion unit 102a converts the planar image P1 acquired in Step SP13 into a voice to generate the audio signal A1 (Step SP17). The first conversion unit 102a outputs the generated voice signal A1 to the communication unit 103. The communication unit 103 superimposes the audio signal A1 on the emergency notification voice and transmits it (Step SP19). The emergency notification start unit 106 ends the series of processes. When the emergency notification start trigger is input from the in-vehicle device or the like while the process depicted in FIG. 12 is being performed, the emergency notification start unit 106 ends the process depicted in FIG. 12.

According to the present embodiment, since an utterance is detected from the voice acquired by the microphone 12 and the audio signal A1 is generated when an utterance is not detected for the predetermined time, the notification device 2 can automatically generate and transmit the audio signal A1 in a situation in which the occupant cannot make a voice call (fainting or the like).

### Third Embodiment

In the second embodiment of the present invention, the audio signal A1 is generated from the planar image P1 when no utterance by a person in the vehicle interior 10a is detected as a trigger, but the trigger for generating the audio signal A1 from the planar image P1 is not limited to this.

The third embodiment of the present invention is an embodiment in which the audio signal A1 is generated from the planar image P1 using abnormality in the biological information as a trigger. Hereinafter, description is made on a notification device 3 according to the third embodiment of the present invention. Note that the same components as those in the first embodiment are denoted by the same reference numerals, and descriptions thereof will be omitted.

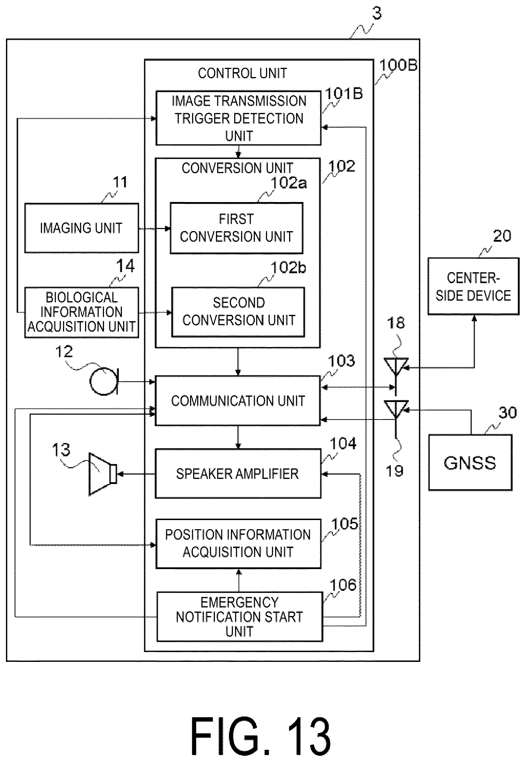

FIG. 13 is a block diagram illustrating an outline of a functional configuration of the notification device 3. Similarly to the notification device 1, the notification device 3 includes the control device 50, the communication device 52, and the storage device 54 as a hardware configuration. The notification device 3 functionally includes a control unit 100B.

The control unit 100B includes at least an image transmission trigger detection unit 101B, the conversion unit 102, the communication unit 103, the speaker amplifier 104, the position information acquisition unit 105, and the emergency notification start unit 106 as software resources by an arithmetic device, such as the Central Processing Unit (CPU) 56 for executing information processing, and the storage device 54.

The image transmission trigger detection unit 101B is a functional unit that detects the image transmission trigger requesting transmission of an image. The biological information acquired by the biological information acquisition unit 14 is input to the image transmission trigger detection unit 101B. In the present embodiment, the biological information acquisition unit 14 is a heart rate meter, and the image transmission trigger detection unit 101B continuously acquires a heart rate.

The image transmission trigger detection unit 101B compares the biological information with a first threshold and a second threshold, and determines that the image transmission trigger is detected when the biological information is smaller than the first threshold or larger than the second threshold. The second threshold is larger than the first threshold. For example, the image transmission trigger detection unit 101B determines that the image transmission trigger is detected when the heart rate is smaller than 50 (first threshold) or when the heart rate is larger than 120 (second threshold).

As described above, in the notification device 3, when the heart rate is smaller than the first threshold or larger than the second threshold, the notification device 3 can automatically determine that the occupant cannot make a voice call (fainting, death, or the like) and a timing to transmit the image of the inside of the vehicle interior 10a has come.

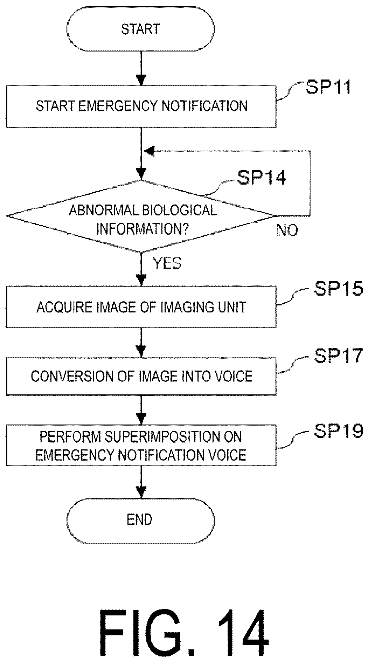

FIG. 14 is a flowchart depicting a flow of processes performed by the notification device 3. First, when the emergency notification start trigger is input, the emergency notification start unit 106 outputs the emergency notification start instruction to the image transmission trigger detection unit 101B, the communication unit 103, the speaker amplifier 104, and the position information acquisition unit 105, and starts the emergency notification (Step SP11).

Next, the image transmission trigger detection unit 101B acquires the biological information from the biological information acquisition unit 14, and determines whether or not there is an abnormality in the biological information, that is, whether or not the biological information is smaller than the first threshold or larger than the second threshold (Step SP14).

When the biological information is neither smaller than the first threshold nor larger than the second threshold (NO in Step SP14), the image transmission trigger detection unit 101B repeats the process in Step SP14. When the biological information is smaller than the first threshold or larger than the second threshold (YES in Step SP14), the image transmission trigger detection unit 101B outputs that the image transmission trigger is input to the conversion unit 102, and the first conversion unit 102a acquires the planar image P1 from the imaging unit 11 (Step SP15).

Next, the first conversion unit 102a converts the planar image P1 acquired in Step SP13 into a voice to generate the audio signal A1 (Step SP17). The first conversion unit 102a outputs the generated voice signal A1 to the communication unit 103. The communication unit 103 superimposes the audio signal A1 on the emergency notification voice and transmits it (Step SP19). The emergency notification start unit 106 ends the series of processes. When the emergency notification start trigger is input from the in-vehicle device or the like while the process depicted in FIG. 14 is being performed, the emergency notification start unit 106 ends the process depicted in FIG. 14.

According to the present embodiment, since the audio signal A1 is generated when the biological information is smaller than the first threshold or larger than the second threshold, the notification device 2 can automatically generate and transmit the audio signal A1 in a situation in which the occupant cannot make a voice call (fainting or the like).

In the present embodiment, the biological information acquisition unit 14 is the heart rate meter, and the image transmission trigger is detected when the biological information falls below the threshold. However, the biological information acquisition unit 14 is not limited to the heart rate meter. For example, the biological information acquisition unit 14 may be a non-contact vital sensor that measures a respiratory frequency or a pulse, a wearable device that measures a heart rate, an arterial oxygen saturation (SpO₂), a body temperature, or the like, or a sphygmomanometer that measures a blood pressure (for example, a watch-type wearable sphygmomanometer). In addition, the image transmission trigger detection unit 101B may determine that the image transmission trigger is detected when, for example, a respiratory frequency, an arterial oxygen saturation, a body temperature, a blood pressure, or the like is smaller than the first threshold or larger than the second threshold according to the acquired biological information.

The embodiments of the invention are described above in detail with reference to the drawings. However, specific configurations are not limited to the embodiments and also include changes in design or the like without departing from the scope of the invention. In addition, the configuration of each of the embodiments described in the present Description can be replaced partially with the configurations of other embodiments.

### Reference Signs List

1, 2, 3: Notification device
10: Vehicle
10a: Vehicle interior
11: Imaging unit
12: Microphone
13: Speaker
14: Biological information acquisition unit
15: Processing unit
18, 19: Antenna
20: Center-side device
21: Microphone
22: Speaker
23: Communication device
24: Processing unit
25: Display unit
26: Antenna
50: Control device
52: Communication device
54: Storage device
56: CPU
58: Memory
100, 100A, 100B: Control unit
101, 101A, 101B: Image transmission trigger detection unit
102: Conversion unit
102a: First conversion unit
102b: Second conversion unit
103: Communication unit
104: Speaker amplifier
105: Position information acquisition unit
106: Emergency notification start unit

## Claims

1. A notification device (1) mounted on a vehicle (10), comprising:
an imaging unit (11) provided in the vehicle configured to acquire a planar image (P1) of an inside of a vehicle interior (10a) of the vehicle; **characterized by**
a conversion unit (102, 102a, 102b) that converts the planar image (P1) into an audio signal in the voice band to generate a first audio signal (A1); and
a communication unit (103) provided in the vehicle and transmitting the first audio signal in the voice band.

2. The notification device (1) according to claim 1, comprising
an image transmission trigger detection unit (101) that detects an image transmission trigger requesting transmission of the planar image (P1), wherein
the conversion unit (102, 102a, 102b) generates the first audio signal (A1) when the image transmission trigger is detected.

3. The notification device (1) according to claim 2, wherein
the image transmission trigger is a sound;
the communication unit (103) receives the sound and outputs the sound to the image transmission trigger detection unit (101); and
the image transmission trigger detection unit determines that the image transmission trigger is detected when the sound is detected.

4. The notification device (1) according to claim 2 or 3, comprising
a microphone (21) provided inside of the vehicle interior (10a), wherein
the image transmission trigger detection unit (101) detects an utterance from a voice acquired by the microphone, and determines that the image transmission trigger is detected when an utterance is not detected for a predetermined time.

5. The notification device (1) according to any one of claims 1 to 4, wherein
the conversion unit (102) configures the planar image in which real numbers are two-dimensionally arrayed as a spectrogram, adds phase information to the spectrogram to generate complex number data in which complex numbers are two-dimensionally arrayed, and performs inverse Fourier transform on the complex number data to generate the first audio signal.

6. The notification device (1) according to claim 5, wherein
the conversion unit (102) randomly generates the phase information using a random number.

7. The notification device (1) according to any one of claims 1 to 6, comprising
a time-series data acquisition unit that acquires time-series data, wherein
the conversion unit (102) converts the time-series data into an audio signal in the voice band to generate a second audio signal; and
the communication unit (102) transmits the second audio signal.

8. The notification device (1) according to claim 7, wherein
the conversion unit (102) performs analog modulation on the time-series data such that a frequency range falls within a voice band to generate the second audio signal.

9. The notification device (1) according to claim 7 or 8, wherein
the time-series data acquisition unit is a biological information acquisition unit (14) that acquires biological information including at least one of a heart rate, a respiratory frequency, a blood pressure, a blood sugar level, a body temperature, and an arterial oxygen saturation; and
the conversion unit (102) converts the biological information into an audio signal in the voice band to generate the second audio signal.

10. The notification device (1) according to any one of claims 2 to 4, comprising
a biological information acquisition unit (14) that acquires biological information including at least one of a heart rate, a respiratory frequency, a blood pressure, a blood sugar level, a body temperature, and an arterial oxygen saturation, wherein
when the biological information is smaller than a first threshold or larger than a second threshold larger than the first threshold, the image transmission trigger detection unit determines that the image transmission trigger is detected.

11. The notification device (1) according to any one of claims 1 to 10, wherein
the imaging unit (11) continuously images the planar image (P1); and
the conversion unit (102) continuously generates the first audio signal (A1).

12. A notification method performed by a notification device as claimed in claims 1-11, the method comprising:
imaging a planar image (P1) of an inside of a vehicle interior (10a) of a vehicle (10);
**characterized by**
converting the planar image into an audio signal in the voice band to generate a first audio signal (A1); and
transmitting the first audio signal.

13. A notification program for causing a computer comprised in a notification device as claimed in claims 1-11 to function as:
a conversion unit (102) that converts a planar image (P1) of an inside of a vehicle interior (10a) imaged by an imaging unit (11) provided in a vehicle (10) into an audio signal in the voice band to generate a first audio signal (A1); and
a communication unit (102) that transmits the first audio signal.

## Patentansprüche

1. Eine an einem Fahrzeug (10) angebrachte Benachrichtigungsvorrichtung (1),
umfassend:
eine im Fahrzeug vorgesehene Bildgebungs-Einheit (11), die dazu aufgebaut ist, ein flächiges Bild (P1) eines Innenraums (10a) des Fahrzeugs zu erfassen; **gekennzeichnet durch**
eine Umwandlungseinheit (102, 102a, 102b), die das ebene Bild (P1) in ein Audiosignal im Sprachband umwandelt, um ein erstes Audiosignal (A1) zu erzeugen; und
eine im Fahrzeug vorgesehene Kommunikationseinheit (103), die das erste Audiosignal im Sprachband überträgt.

2. Benachrichtigungsvorrichtung (1) nach Anspruch 1, umfassend
eine Bildübertragungsauslöser-Erfassungseinheit (101), die einen Bildübertragungsauslöser erfasst, der die Übertragung des flächigen Bildes (P1) anfordert, wobei
die Umwandlungseinheit (102, 102a, 102b) das erste Audiosignal (A1) erzeugt, wenn der Bildübertragungsauslöser erfasst wird.

3. Benachrichtigungsvorrichtung (1) nach Anspruch 2, wobei
der Bildübertragungsauslöser ein Ton ist;
die Kommunikationseinheit (103) den Ton empfängt und den Ton an die Bildübertragungsauslöser-Erfassungseinheit (101) ausgibt; und
die Bildübertragungsauslöser-Erfassungseinheit feststellt, dass der Bildübertragungsauslöser erfasst wurde, wenn der Ton erfasst wird.

4. Benachrichtigungsvorrichtung (1) nach Anspruch 2 oder 3, umfassend
ein Mikrofon (21), das im Fahrzeuginnenraum (10a) vorgesehen ist, wobei
die Bildübertragungsauslöser-Erkennungseinheit (101) eine Äußerung einer vom Mikrofon erfassten Stimme erkennt und feststellt, dass der Auslöser für die Bildübertragung erkannt wurde, wenn über einen vorbestimmten Zeitraum keine Äußerung erkannt wird.

5. Benachrichtigungsvorrichtung (1) nach einem der Ansprüche 1 bis 4, wobei
die Umwandlungseinheit (102) das ebene Bild, in dem reelle Zahlen zweidimensional angeordnet sind, als Spektrogramm aufbaut, dem Spektrogramm Phaseninformationen hinzufügt, um Daten komplexer Zahlen zu erzeugen, in denen komplexe Zahlen zweidimensional angeordnet sind, und eine inverse FourierTransformation für die Daten komplexer Zahlen durchführt, um das erste Audiosignal zu erzeugen.

6. Benachrichtigungsvorrichtung (1) nach Anspruch 5, wobei
die Umwandlungseinheit (102) die Phaseninformationen unter Nutzung einer Zufallszahl zufällig erzeugt.

7. Benachrichtigungsvorrichtung (1) nach einem der Ansprüche 1 bis 6,
umfassend
eine Zeitreihendaten-Erfassungseinheit, die Zeitreihendaten erfasst, wobei
die Umwandlungseinheit (102) die Zeitreihendaten in ein Audiosignal im Sprachband umwandelt, um ein zweites Audiosignal zu erzeugen; und
die Kommunikationseinheit (102) das zweite Audiosignal überträgt.

8. Benachrichtigungsvorrichtung (1) nach Anspruch 7, wobei
die Umwandlungseinheit (102) eine analoge Modulation der Zeitreihendaten so durchführt, dass ein Frequenzbereich innerhalb eines Sprachbands liegt, um das zweite Audiosignal zu erzeugen.

9. Benachrichtigungsvorrichtung (1) nach Anspruch 7 oder 8, wobei
die Zeitreihendaten-Erfassungseinheit eine Erfassungseinheit (14) für biologische Informationen ist, die biologische Informationen erfasst, die mindestens einen der folgenden Werte umfassen: Herzfrequenz, Atemfrequenz, Blutdruck, Blutzuckerspiegel, Körpertemperatur und arterielle Sauerstoffsättigung; und
die Umwandlungseinheit (102) die biologischen Informationen in ein Audiosignal im Sprachband umwandelt, um das zweite Audiosignal zu erzeugen.

10. Benachrichtigungsvorrichtung (1) nach einem der Ansprüche 2 bis 4, umfassend
eine Erfassungseinheit für biologische Informationen (14), die biologische Informationen erfasst, die mindestens einen der folgenden Werte umfassen: Herzfrequenz, Atemfrequenz, Blutdruck, Blutzuckerspiegel, Körpertemperatur und arterielle Sauerstoffsättigung, wobei
wenn die biologischen Informationen kleiner als ein erster Schwellenwert oder größer als ein zweiter Schwellenwert sind, der größer als der erste Schwellenwert ist, die Erfassungseinheit für den Bildübertragungsauslöser bestimmt, dass der Bildübertragungsauslöser erkannt wurde.

11. Benachrichtigungsvorrichtung (1) nach einem der Ansprüche 1 bis 10, wobei
die Bildgebungs-Einheit (11) kontinuierlich das ebene Bild (P1) aufnimmt; und
die Umwandlungseinheit (102) kontinuierlich das erste Audiosignal (A1) erzeugt.

12. Benachrichtigungsverfahren, das von einer in den Ansprüchen 1 bis 11 beanspruchten Benachrichtigungsvorrichtung durchgeführt wird, wobei das Verfahren umfasst:
Aufnehmen eines flächigen Bildes (P1) eines Innenraums (10a) eines Fahrzeugs (10);
**gekennzeichnet durch**:
Umwandeln des flächigen Bildes in ein Audiosignal im Sprachband, um ein erstes Audiosignal (A1) zu erzeugen; und
Senden des ersten Audiosignals.

13. Benachrichtigungsprogramm, das bewirkt, dass ein Computer, der in einer in den Ansprüchen 1-11 beanspruchten Benachrichtigungsvorrichtung enthalten ist, als Folgendes arbeitet:
eine Umwandlungseinheit (102), die ein flächiges Bild (P1) des Innenraums (10a) eines Fahrzeugs, das von einer in einem Fahrzeug (10) vorgesehenen Abbildungseinheit (11) aufgenommen wurde, in ein Audiosignal im Sprachband umwandelt, um ein erstes Audiosignal (A1) zu erzeugen; und
eine Kommunikationseinheit (102), die das erste Audiosignal überträgt.

## Revendications

1. Dispositif de notification (1) monté sur un véhicule (10), comprenant :
une unité de formation d'image (11) disposée dans le véhicule et configurée pour acquérir une image plane (P1) de l'espace intérieur d'un habitacle (10a) de véhicule ; **caractérisé par**
une unité de conversion (102, 102a, 102b) qui convertit l'image plane (P1) en un signal audio dans la bande vocale pour générer un premier signal audio (A1) ; et
une unité de communication (103) disposée dans le véhicule et émettant le premier signal audio dans la bande vocale.

2. Dispositif de notification (1) selon la revendication 1, comprenant
une unité de détection de déclencheur de transmission d'image (101) qui détecte un déclencheur de transmission d'image demandant la transmission de l'image plane (P1), dans lequel
l'unité de conversion (102, 102a, 102b) génère le premier signal audio (A1) lorsque le déclencheur de transmission d'image est détecté.

3. Dispositif de notification (1) selon la revendication 2, dans lequel
le déclencheur de transmission d'image est un son ;
l'unité de communication (103) reçoit le son et fournit le son à l'unité de détection de déclencheur de transmission d'image (101) ; et
l'unité de détection de déclencheur de transmission d'image détermine que le déclencheur de transmission d'image est détecté lorsque le son est détecté.

4. Dispositif de notification (1) selon la revendication 2 ou 3, comprenant
un microphone (21) disposé dans l'habitacle (10a) de véhicule, dans lequel
l'unité de détection de déclencheur de transmission d'image (101) détecte une énonciation d'une voix acquise par le microphone et détermine que le déclencheur de transmission d'image est détecté lorsqu'aucune énonciation n'est détectée pendant un temps prédéterminé.

5. Dispositif de notification (1) selon l'une quelconque des revendications 1 à 4, dans lequel
l'unité de conversion (102) configure l'image plane dans laquelle des nombres réels sont ordonnés en deux dimensions sous forme de spectrogramme, ajoute des informations de phase au spectrogramme pour générer des données de nombres complexes dans lesquelles des nombres complexes sont ordonnés en deux dimensions et effectue une transformée de Fourier inverse sur les données de nombres complexes pour générer le premier signal audio.

6. Dispositif de notification (1) selon la revendication 5, dans lequel
l'unité de conversion (102) génère aléatoirement les informations de phase à l'aide d'un nombre aléatoire.

7. Dispositif de notification (1) selon l'une quelconque des revendications 1 à 6, comprenant
une unité d'acquisition de données chronologiques qui acquiert des données chronologiques, dans lequel
l'unité de conversion (102) convertit les données chronologiques en un signal audio dans la bande vocale pour générer un second signal audio ; et
l'unité de communication (102) émet le second signal audio.

8. Dispositif de notification (1) selon la revendication 7, dans lequel
l'unité de conversion (102) effectue une modulation analogique sur les données chronologiques de sorte qu'une plage de fréquences ne tombe dans une bande vocale pour générer le second signal audio.

9. Dispositif de notification (1) selon la revendication 7 ou 8, dans lequel
l'unité d'acquisition de données chronologiques est une unité d'acquisition d'informations biologiques (14) qui acquiert des informations biologiques incluant au moins l'une parmi une fréquence cardiaque, une fréquence respiratoire, une pression artérielle, une glycémie, une température corporelle et une saturation artérielle en oxygène ; et
l'unité de conversion (102) convertit les informations biologiques en un signal audio dans la bande vocale pour générer le second signal audio.

10. Dispositif de notification (1) selon l'une quelconque des revendications 2 à 4, comprenant
une unité d'acquisition d'informations biologiques (14) qui acquiert des informations biologiques incluant au moins l'une parmi une fréquence cardiaque, une fréquence respiratoire, une pression artérielle, une glycémie, une température corporelle et une saturation artérielle en oxygène, dans lequel
lorsque les informations biologiques sont inférieures à un premier seuil ou supérieures à un second seuil supérieur au premier seuil, l'unité de détection de déclencheur de transmission d'image détermine que le déclencheur de transmission d'image est détecté.

11. Dispositif de notification (1) selon l'une quelconque des revendications 1 à 10, dans lequel
l'unité de formation d'image (11) met continuellement en image l'image plane (P1) ; et
l'unité de conversion (102) génère en continu le premier signal audio (A1).

12. Procédé de notification mis en œuvre par un dispositif de notification selon les revendications 1-11, le procédé comprenant :
la mise en image d'une image plane (P1) d'un espace intérieur d'un habitacle (10a) de véhicule d'un véhicule (10) ;
**caractérisé par**
la conversion de l'image plane en un signal audio dans la bande vocale pour générer un premier signal audio (A1) ; et
l'émission du premier signal audio.

13. Programme de notification pour amener un ordinateur intégré à un dispositif de notification, tel que revendiqué dans les revendications 1-11, à fonctionner en tant que :
une unité de conversion (102) qui convertit une image plane (P1) d'un espace intérieur d'un habitacle (10a) de véhicule mis en image par une unité de formation d'image (11), disposée dans un véhicule (10), en un signal audio dans la bande vocale pour générer un premier signal audio (A1) ; et
une unité de communication (102) qui émet le premier signal audio.
